# EUROPEAN PATENT APPLICATION

(11) **EP 4 755 369 A1**
(43) Date of publication of application: **10.06.2026**
(21) Application number: 25212427.6
(22) Date of filing: 30.10.2025
(51) Int. Cl.: A61K 9/00, A61K 9/06, A61K 31/573, A61K 47/02, A61K 47/10, A61K 47/32

(54) **GEL FORMULATION**

(30) Priority: 04.12.2024 IT 202400027450
(71) Applicant: Esapharma S.p.A., 22066 Melzo (MI) (IT)
(72) Inventor: PEREGO, Massimiliano, Monza MB (IT)
(74) Representative: Longoni, Alessandra

(57) **Abstract**

A gel formulation is described, in particular a gel formulation that can be used as a "base" formulation for various active ingredients, characterized by a specific qualitative and quantitative combination of monopropylene glycol and high-viscosity carbomer. The gel formulation also contains an acidity neutralizing agent and may contain an opacifier and a colorant and/or fragrance. It is free of alcohol and preservatives and is particularly suitable for the formulation of lipophilic and/or poorly water-soluble active ingredients for topical use.

## Description

The present invention relates to a gel formulation, in particular a gel formulation that can be used as a "base" formulation for various active ingredients, characterized by a specific qualitative and quantitative combination of monopropylene glycol and high-viscosity carbomer.

### Background to the invention

Skin conditions can be treated systemically or by applying pharmaceutical forms specifically designed for local application. The issue of comorbidity and therefore the need to use several drugs simultaneously, in addition to the presence of side effects, often leads to a preference for local treatment.

Formulations for external use are used to deliver numerous active ingredients to the skin. These formulations can be ointments, but due to their occlusive power and greasiness, creams and emulsions are generally preferred.

Among creams, some "universal" or "base" formulations have been developed, often derived from cosmetics, which can be used to deliver different active ingredients and treat different conditions. Examples of base creams include Beeler's base cream, composed of cetyl alcohol, beeswax, polyethylene glycol, and sodium lauryl sulfate, and Lanette base cream, containing Lanette wax with triethanolamine and Cetiol (din-octyl carbonate).

However, formulations of this type require the use of emulsifiers (often lecithins, sodium salts of long-chain alcohols, or sulfonates) to lower the surface tension and make them more stable. By their very nature, emulsions tend to separate over time into their two component phases, lipophilic and hydrophilic.

In addition, it should be noted that very often the raw materials of the fatty phase are solid at room temperature. For example, cetyl alcohol has a melting point of 46°C and beeswax has a melting point above 60°C. From a production point of view, therefore, it is advisable to combine all the lipophilic excipients, heat them to obtain a semi-solid mass that can be worked, and then add them at a later stage to the aqueous phase, which is also preheated to prevent immediate precipitation of the emulsion. For this reason, in the preparation of these formulations, it is now preferable to use lipophilic excipients with a lower melting point than the previous ones, also to ensure greater stability of the active ingredients, which are frequently insoluble in water and therefore dissolved in the lipophilic phase.

The evolution of pharmaceutical technology has led to the development of emulsion formulations with characteristics that are increasingly able to preserve the characteristics of the active ingredients they carry, particularly due to their susceptibility to high temperatures. These formulations are water-in-oil-in-water (W/O/W) or oil-in-water-in-oil (O/W/O) emulsions.

An alternative to creams and emulsions is gels, which are dispersions characterized by greater stability and less greasiness.

One of the commonly used excipients in gels is monopropylene glycol.

WO2010094806 (Abigo Medical AB) describes a clobetasol propionate formulation containing approximately 5% w/w propylene glycol as a solvent and moisture-retaining agent, and macrogol glycerol hydroxystearate as a nonionic emulsifier. The main component of such formulations is water (70-85% w/w). The formulations allow formulations with a very low amount (<0.05% w/w) of active ingredient to be prepared. US20160303154 (Gavis Pharmaceuticals) refers to a topical ivermectin gel comprising a glycol, a gelling agent, and water. The amount of glycol is at least 45% by weight. The exemplary formulations contain 53-55% by weight of propylene glycol and 1% by weight of hypromellose. The formulation overcomes the problems associated with the incompatibility of ivermectin with numerous excipients and contact with water.

WO2021158560 (Taro Pharmaceutical) refers to a topical formulation of montelukast. The formulation described can be in the form of an emulsion containing 0.4-4% by weight of carbomer or in the form of a gel containing 80-95% by weight of an amphiphilic compound, preferably propylene glycol, and 0.2-8% by weight of cellulose polymer. The formulations are chemically and physically stable, in particular they contain a very low amount of the known impurities of montelukast (sulfoxide, diol, methyl styrene, cis isomer, Michael adducts, and methyl ketone impurities).

The formulations described in the prior art are generally gels suitable for the formulation of a specific active ingredient.

To our knowledge, there is no "base" gel formulation that can be used for different active ingredients.

### Summary of the invention

We have now found that it is possible to obtain a "base" gel formulation using a specific qualitative and quantitative combination of monopropylene glycol and high-viscosity carbomer. More specifically, the gel formulation of the present invention has as its main component monopropylene glycol in combination with small amounts of a high-viscosity carbomer as a gelling agent, specifically carbomer homopolymer C. The gel formulation of the invention is also alcohol-free and has a low water content.

### Description of the figures

Fig. 1 shows the permeation flow into the skin of the active ingredient of a gel according to the invention compared to a cream.
Fig. 2 shows a comparison between the amount of active ingredient permeated into the skin using a gel according to the invention and a cream.

### Detailed description of the invention

Object of the present invention is a gel formulation characterized by consisting of at least 70% by weight of monopropylene glycol, 0.1-1% by weight of carbomer homopolymer C, 0-1% by weight of titanium dioxide, 0.01-0.1% by weight of acidity neutralizing agent, and water.

The formulation object of the present invention has monopropylene glycol as its main component and contains small amounts of carbomer homopolymer C as a gelling agent.

An acidity neutralizing agent, preferably sodium hydroxide in solution, is added to the formulation to neutralize the carbomer homopolymer C, in order to neutralize its acidity and increase the viscosity of the liquid mass.

The formulation can also optionally contain titanium dioxide as an opacifier and light reflector to protect the active ingredient from photosensitivity reactions.

The gel according to the invention has a low water content (<30% by weight) and contains no alcohol, surfactants, or preservatives.

A colorant and/or fragrance can be optionally added to the gel for the sole purpose of improving the aesthetic and organoleptic characteristics of the final formulation.

The gel formulation object of the present invention can potentially be used to deliver numerous active ingredients for topical use, preferably but not exclusively lipophilic and/or poorly water-soluble active ingredients. Without wishing to be bound by this parameter, the active ingredients that can be used in the gel formulation of the present invention have an octanol/water partition coefficient KR (log P) greater than 0.1.

The formulation can contain one or more active ingredients, generally in an amount by weight between 0.001% and 10%, more preferably between 0.01 and 5%.

The active ingredients can be anti-inflammatory, analgesic, antibacterial, antibiotic, antiseptic, local anesthetic, antifungal, antitumor, antioxidant, muscle relaxant, antihistamine, and antiviral agents for topical use, generally already commercially available in the form of a cream (emulsion).

Specific non-limiting examples of active ingredients that can be used in the gel formulation of the present invention are: tocopherols, terbinafine, prilocaine, lidocaine, desonide, acyclovir, azelaic acid, hydrocortisone, metocarbamol, promethazine, fenticonazole, enoxolone, quinisocaine or dimethisoquin, betamethasone, permethrin, tetracaine, salicylic acid, econazole, bifonazole, docosanol, alprostadil, diflucortolone, isoconazole, itraconazole, fusidic acid, sulfadiazine, ciclopirox, mometasone, ibuprofen, fluticasone, oxiconazole, chlorhexidine, difluprednate, idrocilamide, cyclomethicaine, adapalene, levulinic acid, promestriene, calcipotriol, ketoconazole, crotamitone, isotipendyl, omoconazole, tretinoin, clotrimazole, clobetasol, fluorouracil, fluconazole, metronidazole, fluocinolone, sertaconazole, triamcinolone, tioconazole, imiquimod, trifarotene, ivermectin, cetrimide, flumetasone, minocycline, cincocaine, nifuratel, nystatin, amcinonide, dithranol or anthralin, penciclovir, prednicarbate, nonivamide, nicoboxil, meclocycline, foscarnet, flufenamic acid, miconazole, etofenamate, methylprednisolone, retinol, dexpanthenol, tolnaftate, prednisolone, fluprednidene, gentamicin, ozenoxacin, fluocinonide, naftifine, amorolfine, levomenol, diphenhydramine, benzidamine, bendazac, thiocolchicoside, oxetacaine, piroxicam, fluocortolone, dexchlorpheniramine, aceclofenac, mupirocin, clobetasone, naproxen, ketoprofen, erythromycin, troxerutin, alcinonide, neomycin, coumarin, nadifloxacin, sildenafil, optionally in salt and/or ester form, where applicable.

Preferably, the gel formulation of the present invention contains an active ingredient selected from retinol, tocopherols, itraconazole, tioconazole, clobetasol, sildenafil, fluconazole, clotrimazole, minocycline, and gentamicin, optionally in salt and/or ester form, where applicable.

In a particularly preferred embodiment, the gel formulation according to the present invention is used as a "base" for an anti-inflammatory active ingredient for topical use. More preferably, the anti-inflammatory active ingredient is a clobetasol ester, in particular clobetasol 17-propionate.

In its particularly preferred embodiment, the formulation object of the present invention contains clobetasol 17-propionate, a synthetic corticosteroid with anti-inflammatory activity, as the active ingredient.

An example of a particularly preferred gel formulation is as follows:

| | |
|---|---|
| Clobetasol 17-propionate | 0.05% (w/w) |
| Monopropylene glycol | 70% (w/w) |
| Carbomer homopolymer C | 0.7608% (w/w) |
| Titanium dioxide | 0.5% (w/w) |
| Sodium hydroxide | 0.044% (w/w) |
| Purified water | q.s. to 100% |

Titanium dioxide is not an essential element of the gel formulation of the present invention as it has only an opacifying and light-reflecting function, protecting the active ingredient from photosensitivity reactions.

Similarly, the gel formulation of the present invention can optionally contain excipients such as colorants and/or fragrances.

The gel formulation of the present invention is prepared by carrying out the following steps:
- mixing of monopropylene glycol and the active ingredient
- adding optional titanium dioxide
- adding any optional colorant in solution
- adding the gelling agent (carbomer homopolymer C)
- adding part of the water
- adding the aqueous sodium hydroxide solution to neutralize the acidity
- adding the remaining amount of water
all processing steps are carried out at a temperature below 35°C, between 10°C and 32°C.

The gel formulation of the present invention has a number of advantages over other topical formulations such as emulsions and creams.
- Highly effective spreadability thanks to the continuous carboxypolymethylene phase
- No bacterial growth even without the presence of preservatives, thanks to the proven bacteriostatic activity of monopropylene glycol
- Long shelf life (3 years at 30°C) compared to the average shelf life of creams (2 years at 30°C)
- Minimal formation of impurities during the production process as it is not necessary to heat to make a lipophilic and a hydrophilic phase coexist, as in emulsions, since the gel formulation is characterized by a single phase
- More economical production process as it requires less energy consumption
- Reduced number of components, resulting in simplification of the stages and times of the production process.

Furthermore, when titanium dioxide is present in the formulation, it remains stable over time in the formulation without separating, which is another advantageous feature of the present invention.

Also in terms of safety, the gels of the invention have advantages over creams, as evidenced by in vitro tests of the permeation of the active ingredient under consideration.

These advantages of the "base" gel formulation according to the present invention are related to the presence of monopropylene glycol in high quantities (≥ 70% by weight) in combination with a specific excipient, carbomer homopolymer C, i.e., a high-viscosity cross-linked acrylic acid polymer (> 40000 cP). The product is commercially available, for example under the trade name Carbopol^{®} 980 NF.

Without wishing to be bound by any theory, the inventors believe that the unique characteristics and advantages of the gel formulation of the present invention are due to the qualitative and quantitative combination of the two components, monopropylene glycol and carbomer homopolymer C, which therefore represents the essential feature of the present invention. This specific combination is also able to optimize the "reservoir" effect of the gels at cutaneous level, giving the formulation a higher release rate of the active ingredient than the cream and greater safety characteristics by limiting the systemic absorption of the active ingredient.

In order to better illustrate the present invention, some examples of embodiments of the invention are given below. These examples are for illustrative purposes only and do not in any way limit the scope of the present invention, which is defined by the accompanying claims.

### Example 1

### Preparation of a clobetasol 17-propionate gel formulation according to the invention

The gel was prepared, maintaining a temperature between 10 and 32°C, by transferring 1,680 kg of monopropylene glycol into the turboemulsifier. The turbine was operated at 900-1000 rpm and the temperature of the monopropylene glycol was constantly checked inside the turboemulsifier to ensure that it was between 10 and 32°C. 1,200 g of clobetasol-17-propionate was added and the solution was kept under stirring with the turbine for 10 min at 900-1000 rpm, then the planetary mixer was operated at a speed of 17-18 rpm for 15 minutes. Next, 12.00 kg of titanium dioxide was added to the mixture being stirred under the conditions indicated above, and the vacuum pump was activated at -0.3/-0.6 atm. The stirring speed of the turbine was increased to 1000-1200 rpm while the planetary mixer remained at a speed of 17-18 rpm. Then, 18.26 kg of Carbomer gelling agent was added to the turboemulsifier, 600 kg of purified water was loaded, and the turbine and anchor agitator were stopped for a correct weight reading.

The turbine and the planetary mixer were restarted at speeds of 1000-1200 rpm and 17-18 rpm, respectively, maintaining the temperature between 10 and 35°C.

The mixture was kept under stirring for 40 minutes.

A solution of 1,066 g of sodium hydroxide dissolved in 30 liters of purified water was prepared and, after natural heating due to dissolution, the solution was brought back to a temperature between 25 and 35°C.

The so prepared solution was added to the stirred mass in about 15-20 minutes, maintaining the temperature of the system between 10 and 32°C. Stirring was maintained with a turbine at 1000-1200 rpm and a planetary mixer at 17-18 rpm, always at a temperature between 10 and 32°C, and purified water was then added as needed to 2400 kg, stopping the stirring to record the exact weight. Stirring was resumed with a turbine at 900-1000 rpm and a planetary mixer at 13-14 rpm for 30 minutes, maintaining the temperature between 10 and 32°C and the pH between 5.0 and 6.0.

The resultant gel formulation has the following composition:

| | |
|---|---|
| Clobetasol 17-propionate | 0.05% (w/w) |
| Monopropylene glycol | 70% (w/w) |
| Carbomer homopolymer C | 0.7608% (w/w) |
| Titanium dioxide | 0.5% (w/w) |
| Sodium hydroxide | 0.044% (w/w) |
| Purified water | q.s. to 100% |

### Example 2

### Release test

The clobetasol 17-propionate gel formulation according to the invention, obtained as described in Example 1, was evaluated by an in vitro release test and an in vitro permeation test according to the guidelines proposed by the EMA (CHMP/QWP/708282/2018).

For the formulation of the invention, the release test showed that the release of clobetasol 17-propionate is greater than 70%.

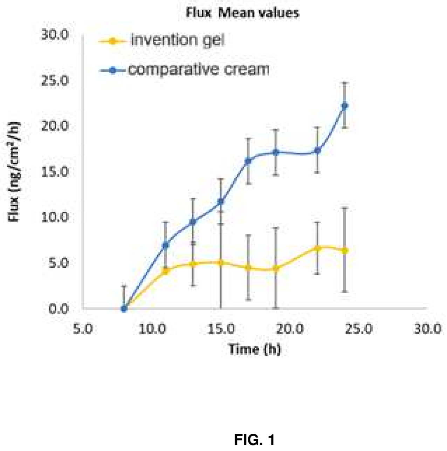

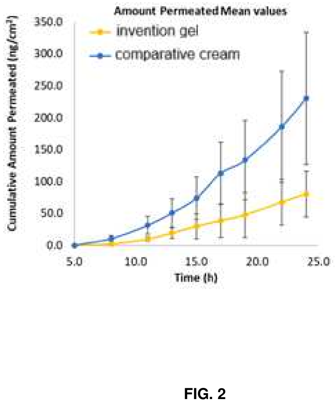

The in vitro permeation test was performed in comparison with a commercial cream product (Clobesol^{®}) and the results are shown graphically in Figures 1 and 2 and numerically in Tables 1 and 2 below.

**Table 1 - Flow (J) in ng/cm²/h of active ingredient in the skin**

| | Gel of the invention (n=3) | | Clobesol^{®} (n=5) | |
|---|---|---|---|---|
| Time (hours) | Mean | RSD% | Mean | RSD% |
| 8 | BLQ | BLQ | N/A | N/A |
| 11 | 4.08 | N/A | 6.97 | 43.01 |
| 13 | 4.90 | 48.76 | 9.53 | 48.03 |
| 15 | 5.06 | 109.66 | 11.75 | 46.48 |
| 17 | 4.49 | 78.77 | 16.15 | 43.87 |
| 19 | 4.46 | 98.57 | 17.11 | 40.96 |
| 22 | 6.63 | 42.24 | 17.37 | 49.94 |
| 24 | 6.43 | 71.63 | 22.26 | 36.74 |
| J_{MAX} | 8.95 | 49.30 | 22.26 | 36.74 |

| | | | | |
|---|---|---|---|---|
| BLQ = below the limit of quantification RSD = Relative Standard Deviation N/A = not available | | | | |

**Table 2 - Amount of permeated active ingredient (AP) in ng/cm²**

| | Gel of the invention (n=3) | | Clobesol^{®} (n=5) | |
|---|---|---|---|---|
| Time (hours) | Mean | RSD% | Mean | RSD% |
| 5 | BLQ | N/A | BLQ | N/A |
| 8 | 2.02 | N/A | 10.55 | 45.40 |
| 11 | 9.82 | 39.21 | 31.46 | 43.62 |
| 13 | 19.62 | 43.76 | 50.52 | 45.08 |
| 15 | 29.73 | 65.61 | 74.02 | 44.97 |
| 17 | 38.71 | 68.46 | 113.01 | 42.86 |
| 19 | 47.63 | 73.95 | 113.56 | 46.38 |
| 22 | 67.52 | 52.91 | 185.65 | 47.00 |
| 24 | 80.38 | 45.15 | 230.18 | 44.95 |
| AP% 24 hours | 2.10 | 1.04 | 4.65 | 44.52 |

| | | | | |
|---|---|---|---|---|
| BLQ = below the limit of quantification RSD = Relative Standard Deviation N/A = not available | | | | |

The data show that the permeation of the active ingredient into the subcutaneous layer and therefore into the bloodstream is much higher for Clobetasol^{®} cream than for the gel formulation according to the invention, confirming the greater safety of the gel for topical use.

The mass balance and dose reproducibility data obtained from the permeation tests also confirm the greater safety of the gel according to the invention compared to the cream.

The data are shown in Table 3

**Table 3 - Mass balance and dose reproducibility for the gel according to the invention and Clobesol^{®}**

| Gel | Rep 1 | Rep 2 | Rep 3 | Rep 4 | Rep 5 | Rep 6 | Mean | RSD% |
|---|---|---|---|---|---|---|---|---|
| Dose (mg) | 9.59 | 7.30 | 7.90 | 7.51 | 9.43 | 7.72 | 8.24 | 12.18 |
| Receiving fluid (AP%) | 6.42 | 9.52 | 6.40 | 7.55 | 6.80 | 7.27 | 7.33 | 15.94 |
| Skin (S%) | 10.58 | 10.96 | 14.24 | 19.03 | 17.55 | 16.91 | 14.88 | 23.81 |
| Formulation (R%) | 59.26 | 62.28 | 62.88 | 58.56 | 55.83 | 50.15 | 58.16 | 8.07 |
| Mass balance % | 76.25 | 82.75 | 83.53 | 85.14 | 80.18 | 50.15 | 80.36 | 5.33 |

| Cream | Rep 1 | Rep 2 | Rep 3 | Rep 4 | Rep 5 | Rep 6 | Mean | RSD% |
|---|---|---|---|---|---|---|---|---|
| Dose (mg) | 8.38 | 7.74 | 9.31 | 9.12 | 6.92 | 7.91 | 8.23 | 10.92 |
| Receiving fluid (AP%) | 5.61 | 2.80 | 5.27 | 5.53 | 7.82 | 7.19 | 5.70 | 30.72 |
| Skin (S%) | 3.85 | 3.61 | 8.45 | 9.54 | 4.66 | 5.80 | 5.99 | 41.40 |
| Formulation (R%) | 53.12 | 68.38 | 56.49 | 57.83 | 62.90 | 60.49 | 59.87 | 8.94 |
| Mass balance % | 62.58 | 74.79 | 70.20 | 72.90 | 75.38 | 73.48 | 71.56 | 6.64 |

## Claims

1. A base gel formulation **characterized by** consisting of at least 70% by weight of monopropylene glycol, 0.1-1% by weight of carbomer homopolymer C, 0-1% by weight of titanium dioxide, 0.01-0.1% by weight of acidity neutralizing agent and water.

2. The formulation according to claim 1 wherein the acidity neutralizing agent is sodium hydroxide.

3. The formulation according to claim 1 and 2 for use in formulating one or more lipophilic and/or poorly water soluble active ingredients.

4. The formulation according to claim 3 wherein the one or more active ingredients are selected from retinol, tocopherols, itraconazole, tioconazole, clobetasol, sildenafil, fluconazole, clotrimazole, minocycline and gentamycin, optionally in salified and/or esterified form, when applicable.

5. The formulation according to claim 4 wherein the active ingredient is clobetasol in its esterified form clobetasol 17-propionate.

6. The formulation according to anyone of claims 3-5 further containing a coloring agent and/or a fragrance.

7. A formulation having the following composition:
| | |
|---|---|
| Clobetasol 17-propionate | 0.05% (w/w) |
| Monopropylene glycol | 70% (w/w) |
| Carbomer homopolymer C | 0.7608% (w/w) |
| Titanium dioxide | 0.5% (w/w) |
| Sodium hydroxide | 0.044% (w/w) |
| Purified water | q.s. to 100% |

8. A process for the preparation of the gel formulation according to anyone of the preceding claims comprising the following steps:
- mixing monopropylene glycol and the active ingredient
- optionally adding titanium dioxide
- optionally adding the coloring agent in solution
- adding the gelling agent carbomer homopolymer C
- adding part of the water
- adding the aqueous solution of sodium hydroxide to neutralize the acidity
- adding the remaining amount of water
all steps being carried out at a temperature below 35°C, between 10°C and 32°C.
